# EUROPEAN PATENT APPLICATION

(11) **EP 4 389 035 A1**
(43) Date of publication of application: **26.06.2024**
(21) Application number: 23211222.7
(22) Date of filing: 21.11.2023
(51) Int. Cl.: A61B 18/04, A61B 18/14, A61B 18/12, A61B 18/00

(54) **ELECTROCAUTERY SUCTION IRRIGATION TUBING DEVICE**

(30) Priority: 01.12.2022 TW 111146160; 25.08.2023 TW 112132113
(71) Applicant: Lagis Enterprise Co., Ltd., 43767 Taichung City (TW)
(72) Inventor: CHANG, Kuo-Yang, 43767 Taichung City (TW); CHEN, Chun-Liang, 43767 Taichung City (TW)
(74) Representative: BSB Patentanwälte Schütte & Engelen Partnerschaft mbB

(57) **Abstract**

An electrocautery suction irrigation tubing device includes a communicating tube (21) which defines a main passage (201), a first passage (202) controlled by a first actuator button (23) to be in fluid communication with the main passage (201), and a second passage (203) controlled by a second actuator button (24) to be in fluid communication with the main passage (201). Asuction irrigation cannula (52) is made of a metal material and integrally formed for passing of electric current, and which has a cannula wall (521) defining a cannula passage (520), and at least one first cutter portion (523) formed on the cannula wall (521). The suction irrigation cannula (52) is operable to perform an electrocautery function to cut body tissues or blood coagulum into smaller pieces so as to smoothly pass through the passages to reduce blockage in the passages.

## Description

The disclosure relates to a surgical instrument, and more particularly to an electrocautery suction irrigation tubing device.

Minimally invasive surgery is carried out through an endoscope and a variety of imaging technology to minimize incisions and reduce bleeding and infection during the surgical operation, and lessen postoperative pain. During the surgical procedure, a suction irrigation cannula is required to remove blood and body fluid as well as apply saline solution to cleanse and keep the surgical site clean.

Referring to FIG. 1, a suction irrigation set disclosed in CN208160792 includes a first tube 11 extending in an up-down direction, an irrigation cannula 12 connected with a lower end of the first tube 11 and in communication with the first tube 11, and a second tube 13 fixed on a left side of the first tube 11 and in communication with the first tube 11. The second tube 13 has an irrigation valve 131 and a suction valve 132 in communication with the suction irrigation cannula 12 and respectively connected with an irrigation hose 14 and a suction hose 15. Through the operation of the irrigation valve 131 and the suction valve 132, saline solution from an irrigation device (not shown) is impelled to flow through the irrigation hose 14 and the suction irrigation cannula 12 for cleansing the surgical site, or blood and body fluid at the surgical site is drawn from the suction irrigation cannula 12 to the suction hose 15.

However, blood coagulum or larger tissues may be drawn and obstruct the suction irrigation cannula 12, and would need to be removed manually from the suction irrigation cannula 12 during the surgical procedure, which increases the risk of surgical complications. Otherwise, the suction irrigation set must be replaced, which results in inconvenience and high costs.

Therefore, an object of the disclosure is to provide an electrocautery suction irrigation tubing device that can alleviate at least one of the drawbacks of the prior art.

According to an aspect of the disclosure, there is provided an electrocautery suction irrigation tubing device according to claim 1.

The suction irrigation cannula is operable to perform an irrigation mode and a fluid suction mode, and also performs an electrocautery function. During the fluid suction mode, electric current is applied to the suction irrigation cannula to heat and cut body tissue or blood coagulum into smaller pieces so as to smoothly pass through the passages to reduce blockage in the passages. The electrocautery suction irrigation tubing device is convenient to operate.

Other features and advantages of the disclosure will become apparent in the following detailed description of the embodiments with reference to the accompanying drawings. It is noted that various features may not be drawn to scale.

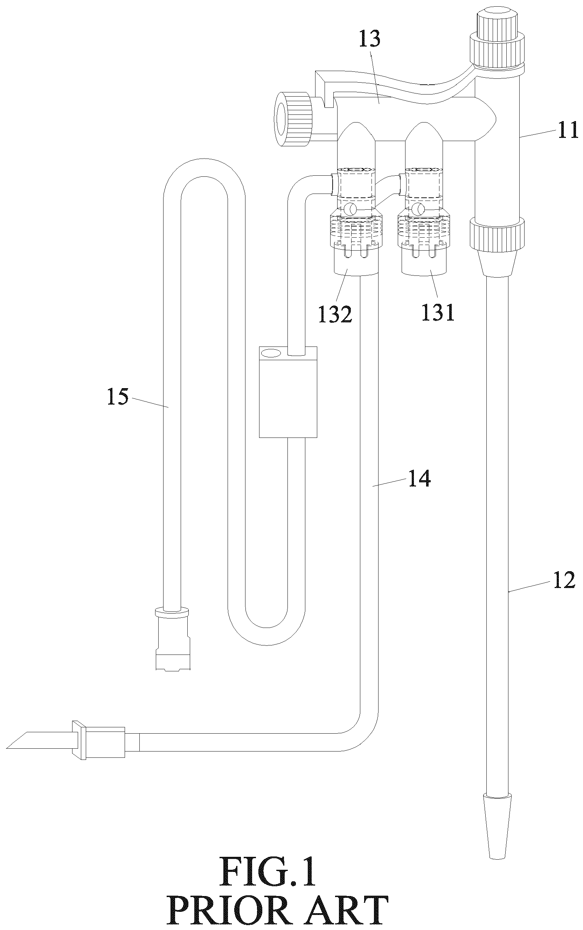

FIG. 1 is a front view illustrating a conventional suction irrigation set disclosed in CN 208160792.
FIG. 2 is a perspective view illustrating a first embodiment of an electrocautery suction irrigation tubing device according to the disclosure.
FIG. 3 is a fragmentary perspective view of the first embodiment.
FIG. 4 is a fragmentary, exploded perspective view illustrating a handle module, an electrocautery suction irrigation module, an irrigation hose and a suction hose of the first embodiment.
FIG. 5 is a fragmentary sectional view illustrating a movable member and a tubular joint of the electrocautery suction irrigation module mounted on the handle module.
FIG. 6 is a sectional view taken from another angle, illustrating an insulation sleeve of the electrocautery suction irrigation module in an electrocautery state relative to an outer tube.
FIG. 7 is a fragmentary perspective view illustrating a suction irrigation cannula of the electrocautery suction irrigation module with first cutter portions and second cutter portions, and the outer tube with a hemostatic portion and two third cutter portions.
FIG. 8 is a sectional view similar to FIG. 6, illustrating the insulation sleeve in a suction irrigation state relative to the outer tube.
FIG. 9 is an enlarged sectional view illustrating the insulation sleeve moved to the electrocautery state relative to the outer tube.
FIG. 10 is an enlarged sectional view illustrating the insulation sleeve moved to the suction irrigation state relative to the outer tube.
FIG. 11 is a sectional view taken along line XI-XI of FIG. 6.
FIG. 12 is a fragmentary sectional view illustrating an insulation sleeve, an outer tube and a suction irrigation cannula of a second embodiment of the disclosure.
FIG. 13 is a fragmentary side view illustrating the suction irrigation cannula of the second embodiment rotated with a rotary disc relative to the outer tube.
FIG. 14 is a fragmentary perspective view illustrating a suction irrigation cannula and an outer tube of a third embodiment of the disclosure.
FIG. 15 is a schematic view illustrating a fourth embodiment of the disclosure in a state of use.
FIG. 16 is a fragmentary front view illustrating a main passage of a handle module of the fourth embodiment in fluid communication with a first passage in a state when a first actuator button is pressed.
FIG. 17 is a view similar to FIG. 16, illustrating the main passage in fluid communication with a second passage in a state when a second actuator button is pressed.
FIG. 18 is a fragmentary perspective view illustrating a suction irrigation cannula and an insulation sleeve of the fourth embodiment.
FIG. 19 is a longitudinal sectional view of FIG. 18.
FIG. 20 is a cross-sectional view of the suction irrigation cannula.

Before the disclosure is described in greater detail, it should be noted that where considered appropriate, reference numerals or terminal portions of reference numerals have been repeated among the figures to indicate corresponding or analogous elements, which may optionally have similar characteristics.

It should be noted herein that for clarity of description, spatially relative terms such as "top," "bottom," "upper," "lower," "on," "above," "over," "downwardly," "upwardly" and the like may be used throughout the disclosure while making reference to the features as illustrated in the drawings. The features may be oriented differently (e.g., rotated 90 degrees or at other orientations) and the spatially relative terms used herein may be interpreted accordingly.

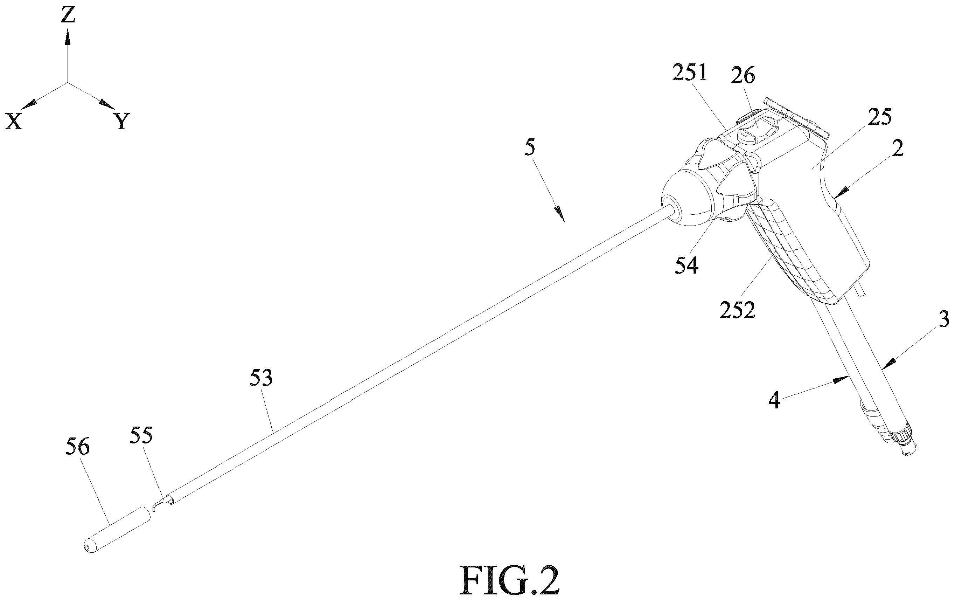

Referring to FIGS. 2 to 4, a first embodiment of an electrocautery suction irrigation tubing device according to the disclosure is utilized with an external irrigator device (not shown), an external suction device (not shown) and an external electrocautery machine (not shown) for surgical operations. The electrocautery suction irrigation tubing device includes a handle module 2, an irrigation hose 3 removably mounted on the handle module 2, a suction hose 4 removably mounted on the handle module 2, and an electrocautery suction irrigation module 5 mounted on the handle module 2 along a front-rear direction (X) and adapted to be electronically connected with the electrocautery machine. The irrigation hose 3 is a flexible pipe and is adapted to be connected with the irrigator device. The suction hose 4 is a flexible pipe and is adapted to be connected with the suction device.

The handle module 2 includes a handgrip 25, a switch button 26 mounted on a horizontal upper portion 251 of the handgrip 25 and operable in an up-down direction (Z) that is transverse to the front-rear direction (X), a communicating tube 21 mounted on the upper portion 251 of the handgrip 25, and a first actuator button 23 and a second actuator button 24 operably mounted outwardly of the handgrip 25. The handgrip 25 further has a gripping portion 252 extending downwardly from and inclined relative to the horizontal upper portion 251. The horizontal upper portion 251 has a front barrel 253. The gripping portion 252 is for gripping by an operator.

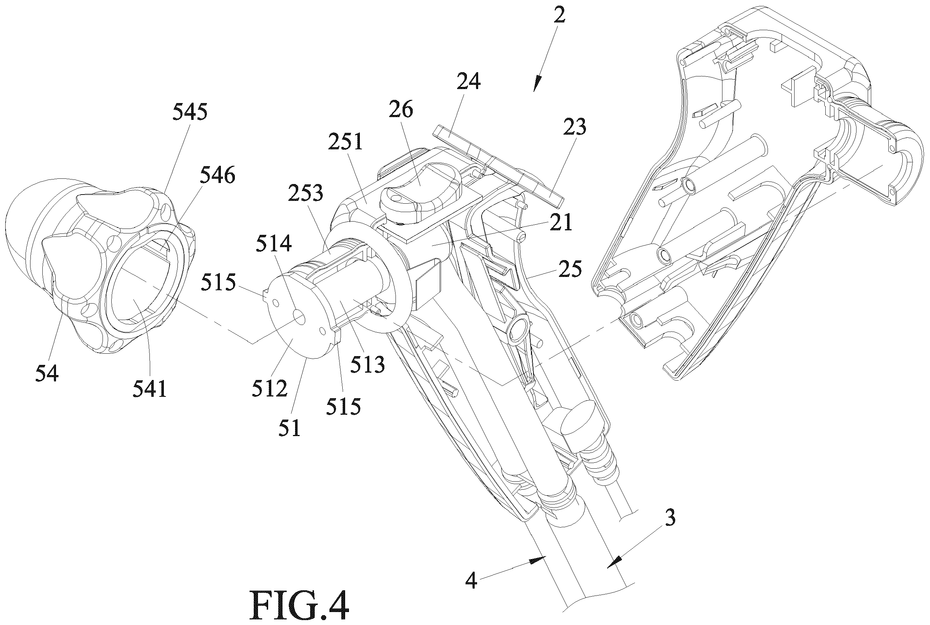

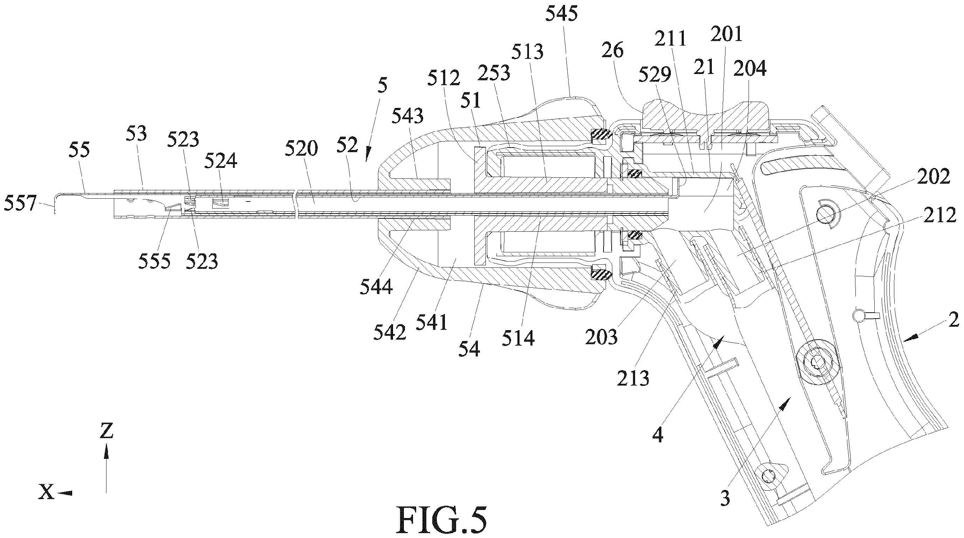

With reference to FIGS. 4 to 6, the communicating tube 21 has a main tube body 211 which extends in the front-rear direction (X) to define a main passage 201, a first tube body 212 which extends downwardly and is inclined rearwardly from the main tube body 211 to define a first passage 202, and a second tube body 213 which is spaced apart from the first tube body 212 and which extends downwardly and is inclined rearwardly from the main tube body 211 to define a second passage 203. The first tube body 212 is connected and cooperates with the irrigation hose 3 to define the first passage 202 which is controlled by the first actuator button 23 to be in fluid communication with the main passage 201. The second tube body 213 is connected and cooperates with the suction hose 4 to define the second passage 203 which is controlled by the second actuator button 24 to be in fluid communication with the main passage 201. The main tube body 211 further has an engaging slot 204 which is confined by left and right vertical plates extending in the front-rear direction (X) (only one vertical plate is shown in FIG. 5).

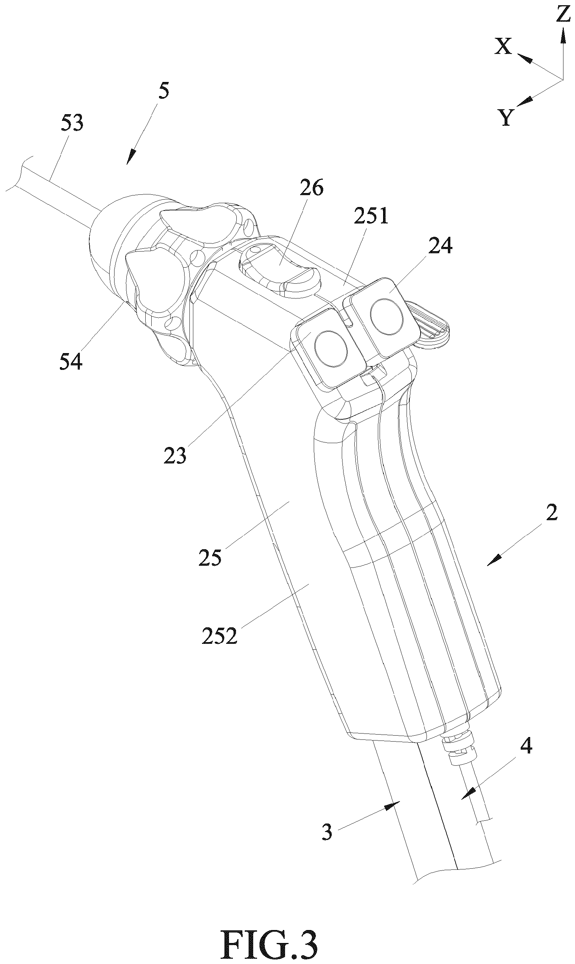

With reference to FIGS. 3 to 5, the first actuator button 23 and the second actuator button 24 are spaced apart from each other in a left-right direction (Y) that is transverse to both the front-rear direction (X) and the up-down direction (Z). When the first actuator button 23 is operated to control the first passage 202 to be in fluid communication with the main passage 201, fluid, such as saline solution from the irrigator device is impelled to enter the irrigation hose 3 and pass through the first passage 202 to flow to the main passage 201 for performing an irrigation mode. When the second actuator button 24 is operated to control the second passage 203 to be in fluid communication with the main passage 201, fluid from the main passage 201 flows to the second passage 203 and to the suction device through the suction hose 4 for performing a fluid suction mode. The term "fluid" here may refer to liquid and gas, and may include liquid with some solid materials (such as blood coagulum or tissues), or gas with some solid material.

With reference to FIGS. 2, 4 and 5, the electrocautery suction irrigation module 5 is connected with the upper portion 251 of the handle module 2, and includes a tubular joint 51 which is mounted on the front barrel 253 of the handle module 2 along the front-rear direction (X) and which is connected with the communicating tube 21, a movable member 54 which movably surrounds the tubular joint 51 and the front barrel 253, a suction irrigation cannula 52 which is inserted into the movable member 54 and the tubular joint 51, an outer tube 55 which is sleeved around the suction irrigation cannula 52, an insulation sleeve 53 which surrounds the outer tube 55, and a protective cap 56 (FIG. 2) which is removably sleeved around a front end of the insulation sleeve 53.

The tubular joint 51 is rotatably mounted on the front barrel 253 and is communicated with the main passage 201 of the communicating tube 21. The tubular joint 51 has a disc-like seat portion 512 abutting against the front barrel 253, a tubular portion 513 extending rearwardly from the seat portion 512 and inserted into the front barrel 253 to be connected with the communicating tube 21, and a tubular hole 514 extending through the seat portion 512 and the tubular portion 513 in the front-rear direction (X) to be in spatial communication with the main passage 201.

With reference to FIGS. 4 to 6, the seat portion 512 has two first engaging features 515 opposite to each other and in the form of protrusions in this embodiment. In various embodiments, the number of the first engaging feature 515 may be only one.

The movable member 54 is rotatably sleeved around the front barrel 253 and is movable relative to the tubular joint 51 and the front barrel 253 in the front-rear direction (X). The movable member 54 is in the form of a rotary knob and has an outer surrounding wall 542 which defines a receiving hole 541, and an inner tubular wall 543 which is radially spaced apart from the outer surrounding wall 542 and which is disposed in the receiving hole 541. A portion of the tubular joint 51 is received in the receiving hole 541. The inner tubular wall 543 defines an inner hole 544 which is formed forwardly of the tubular joint 51.

The outer surrounding wall 542 has an operating portion 545 for gripping by the operator, and two second engaging features 546 which are formed in the receiving hole 541. The second engaging features 546 are slidably engaged with the first engaging features 515 of the tubular joint 51 such that the tubular joint 51 is rotated with the movable member 54 relative to the front barrel 253. In this embodiment, each second engaging feature 546 is in the form of a slot extending in the front-rear direction (X) and having a length larger than that of the corresponding first engaging feature 515 to permit the movement of the movable member 54 relative to the tubular joint 51 in the front-rear direction (X).

With reference to FIGS. 5 to 7, the suction irrigation cannula 52 is made of a metal material and integrally formed to be a single piece, and for introducing saline solution to cleanse the surgical site, or remove blood, body fluid, blood coagulum and tissues from the surgical site. The rear end of the suction irrigation cannula 52 is inserted into the movable member 54 and the tubular joint 51, and securely connected with the communicating tube 21 to be in fluid communication with the main passage 201. The suction irrigation cannula 52 has a cannula wall 521 which defines a cannula passage 520, a plurality of first cutter portions 523 which are formed on the cannula wall 521 and at a front end thereof distal from the handle module 2 and which are bent and extend in the cannula passage 520, a plurality of second cutter portions 524 which are formed on the cannula wall 521 and which are bent and extend in the cannula passage 520, and a hook portion 529 which extends upwardly from a rear end edge of the cannula wall 521 to be engaged in the engaging slot 204 of the communicating tube 21. The shape of the second cutter portions 524 is different from that of the first cutter portions 523. In this embodiment, the first cutter portions 523 are formed at a front end edge of the cannula wall 521 and are spaced apart from each other in a circumferential direction. The second cutter portions 524 are spaced apart from each other in both the circumferential direction and the front-rear direction (X).

The suction irrigation cannula 52 is made by a single tube without any soldering or adhesives, and has a great structural strength, and the cross-section of the cannula passage 520 can be increased for increasing the flow thereof.

The outer tube 55 is inserted, together with the suction irrigation cannula 52, into the movable member 54 and the tubular joint 51. The rear end of the outer tube 55 is securely inserted in the tubular hole 514 of the tubular joint 51 to have the rear end edge be flush with the rear end edge of the tubular hole 514. The outer tube 55 is made of a metal material and integrally formed to be a single piece, and directly contacts with the suction irrigation cannula 52. The electric current from the electrocautery machine flows through the outer tube 55 and the suction irrigation cannula 52. The outer tube 55 has outer and inner tubular surfaces 552, 551 radially opposite to each other. The outer tube 55 further has a hemostatic portion 553 which extends away from the handle module 2 and beyond the suction irrigation cannula 52, a sleeve section 554 which is sleeved around to cover the suction irrigation cannula 52, and two third cutter portions 555 which interconnect the sleeve section 554 and the hemostatic portion 553, which are spaced apart from each other and which are bent and extend radially and inwardly toward the inner tubular surface 551. The hemostatic portion 553 has an elongated section 556 which is connected with the third cutter portions 555, and a front tip end 557 which extends from the elongated section 556 in the up-down direction (Z). In this embodiment, the front tip end 557 extends upwardly from the elongated section 556. The third cutter portions 555 are formed adjacent to but not superimposed upon the first cutter portions 523 of the suction irrigation cannula 52 in the front-rear direction (X). The hemostatic portion 553 is proximal to the first cutter portions 523 and remote from the second cutter portions 524.

The insulation sleeve 53 is in the form of a tube and is inserted, together with the outer tube 55 and the suction irrigation cannula 52, into the movable member 54. The rear end of the insulation sleeve 53 is secured in the inner hole 544 of the movable member 54 so as to be rotated or forward-rearward moved along with the movable member 54.

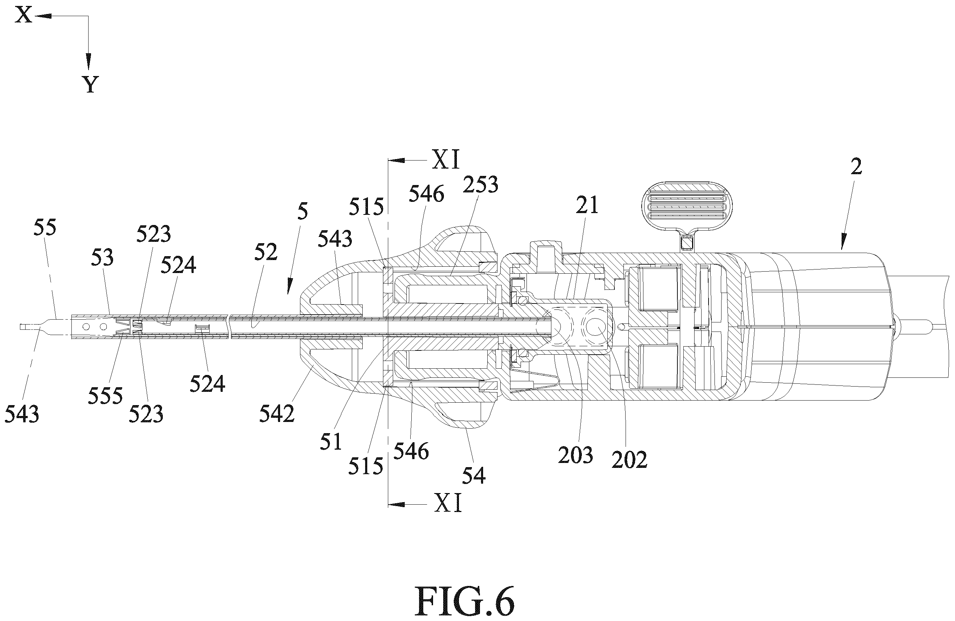

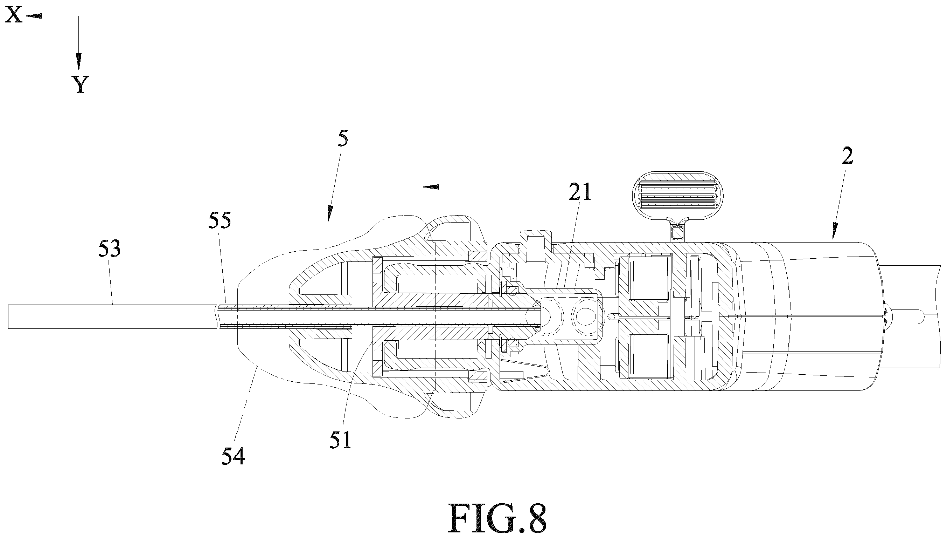

With reference to FIGS. 6 and 8, the movable member 54 is operated to move the insulation sleeve 53 relative to the tubular joint 51 and the outer tube 55 such that the insulation sleeve 53 is movable relative to the outer tube 55 between an electrocautery state (see FIG. 6) and a suction irrigation state (see FIG. 8). When the movable member 54 is moved, the second engaging features 546 slide relative to the first engaging features 515 so as to stabilize the movement of the insulation sleeve 53 between the electrocautery state and the suction irrigation state.

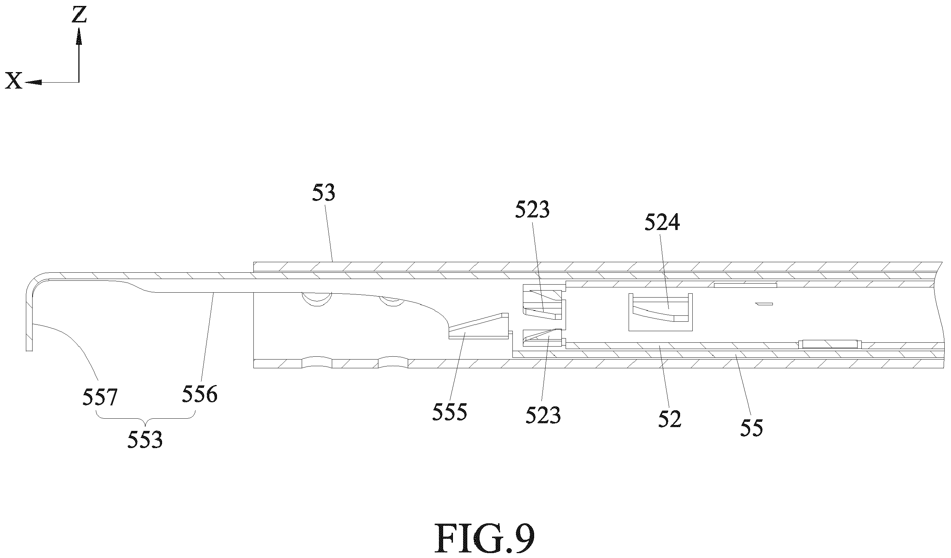

With reference to FIGS. 6 and 9, in the electrocautery state, at least a portion of the hemostatic portion 553 of the outer tube 55 projects from the insulation sleeve 53, and the front ends of the second engaging features 546 abut against the first engaging features 515.

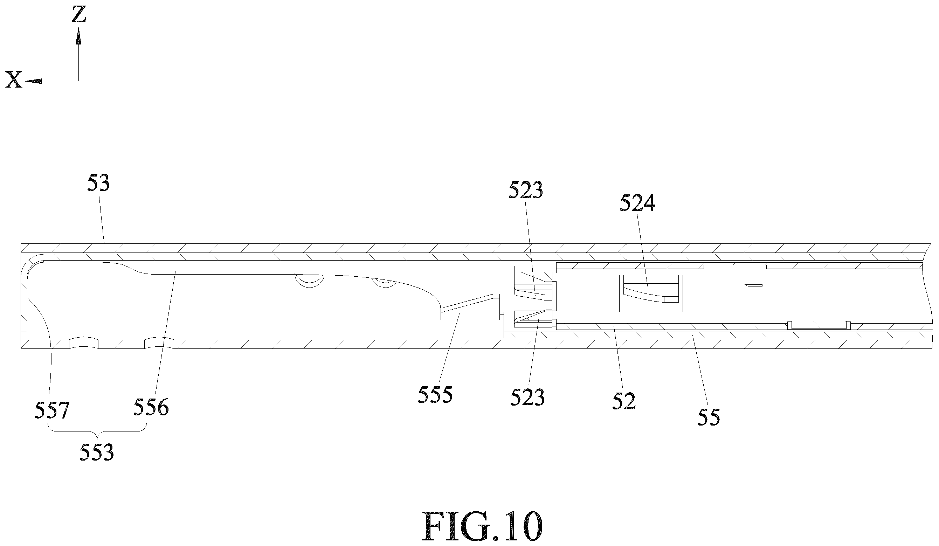

With reference to FIGS. 8 and 10, in the suction irrigation state, the hemostatic portion 553 is covered and concealed by the insulation sleeve 53.

With reference to FIGS. 3, 8 and 10, in this embodiment, the movable member 54 is moved forwardly to the suction irrigation state such that the insulation sleeve 53 is moved forwardly to cover the hemostatic portion 553. In this state, the operator directs the insulation sleeve 53 close to the surgical site to be cleansed, and pushes the first actuator button 23 to perform the irrigation procedure. Alternatively, the second actuator button 24 is pushed to perform the fluid suction procedure. The procedure is stopped when a corresponding one of the first actuator button 23 and the second actuator button 24 is released.

With reference to FIGS. 3, 5 and 6, the movable member 54 is moved rearwardly to expose the hemostatic portion 553 and move the insulation sleeve 53 to the electrocautery state. In this state, the operator pushes the front end of the switch button 26 to control passing of a large electric current from the electrocautery machine into the outer tube 55 and the suction irrigation cannula 52, and actuates an electrocauterization function to cut blood coagulum and tissues. Meanwhile, the second actuator button 24 is pushed to perform the fluid suction procedure for removing the blood, blood coagulum and tissues. Also, through the third cutter portions 555, the first cutter portions 523 and the second cutter portions 524, the blood coagulum and larger tissues are heated and cut into smaller pieces so as to smoothly pass through the passages to reduce blockage in the passages.

Once bleeding occurs during the surgery, the front tip end 557 of the outer tube 55 is pressed against the site of bleeding, and the rear end of the switch button 26 is pushed to actuate the electrocautery machine to pass a small electric current into the outer tube 55 and the suction irrigation cannula 52. An electrocoagulation hemostasis procedure is performed through the resistance and heat generated as a result of the front tip end 557 contacting the body tissues. With the elongated section 556, the front tip end 557 is disposed remote from the third cutter portions 555 so as not to be interfered by the third cutter portions 555, the first cutter portions 523 or the insulation sleeve 53.

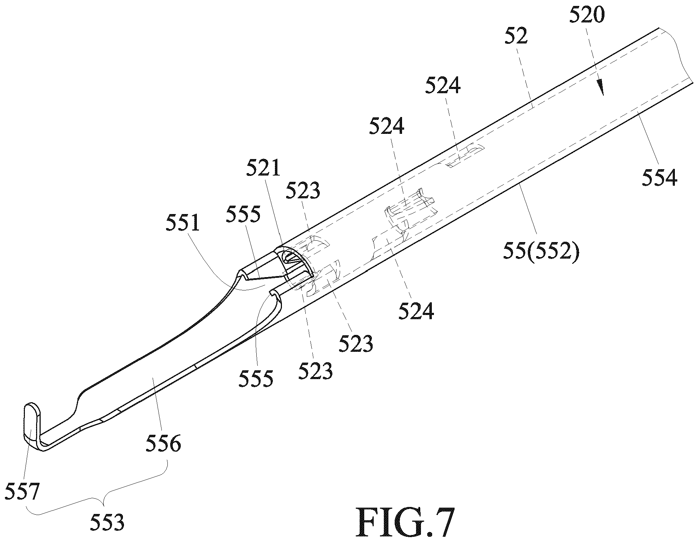

With reference to FIG. 7, the front tip end 557 is also utilized as a hook to manipulate body tissue and facilitate the electrocautery cutting procedure.

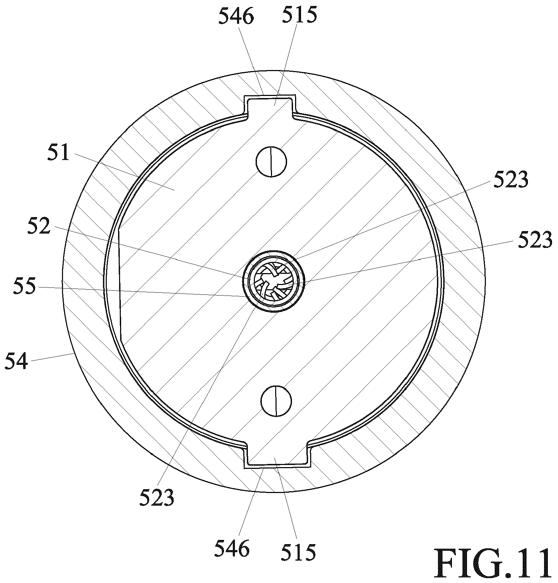

With reference to FIGS. 5, 6 and 11, moreover, in this embodiment, through rotation of the operating portion 545 of the movable member 54 relative to the front barrel 253 of the handle module 2, the outer tube 55 is rotated relative to the suction irrigation cannula 52 to further break up the body tissues and blood coagulum so as to increase the cutting efficiency. It is noted that, since the second engaging features 546 of the movable member 54 engage with the first engaging features 515 of the tubular joint 51, and the outer tube 55 is secured to the tubular joint 51, the tubular joint 51 is rotated with the movable member 54 to bring the outer tube 55 to rotate relative to the suction irrigation cannula 52. With the hook portion 529 engaged in the engaging slot 204 of the communicating tube 215, the suction irrigation cannula 52 is not rotated. Furthermore, in this embodiment, one first engaging feature 515 has a width which is larger than the other first engaging feature 515 for a foolproof design.

Further, in this embodiment, the operator moves the movable member 54 with his or her index and middle fingers gripping on upper and lower sides of the movable member 54 to shift the movable member 54 between the electrocautery state and the suction irrigation state. In the electrocautery state, the operator pushes the front end or the rear end of the switch button 26 with the index finger to actuate an electrocauterization procedure or an electrocoagulation hemostasis procedure. Also, the first actuator button 23 or the second actuator button 24 is pushed with a thumb to perform an irrigation procedure or a fluid suction procedure. Therefore, the electrocautery suction irrigation tubing device of this embodiment is conveniently operable and simple to use in one hand.

Furthermore, the suction irrigation cannula 52 is operated in the irrigation and suction states, and also carries electrocauterization and electrocoagulation modes. That is, during the fluid suction procedure for removing blood coagulum and body tissues, with the first cutter portions 523 and the second cutter portions 524, the blood coagulum and larger tissues are cut into smaller pieces so as to smoothly pass through the passages to reduce blockage in the passages. It is noted that the suction irrigation cannula 52 may only have one first cutter portion 523 and one second cutter portion 524 to perform the electrocauterization procedure. Alternatively, the number of the first and second cutter portions 523, 524 may be increased to increase electrocauterization efficiency. The positions of the first and second cutter portions 523, 524 may be exchanged, modified or alternated.

Moreover, with the outer tube 55 rotated relative to the suction irrigation cannula 52, the body tissues and blood coagulum is further broken up so as to increase the cutting efficiency.

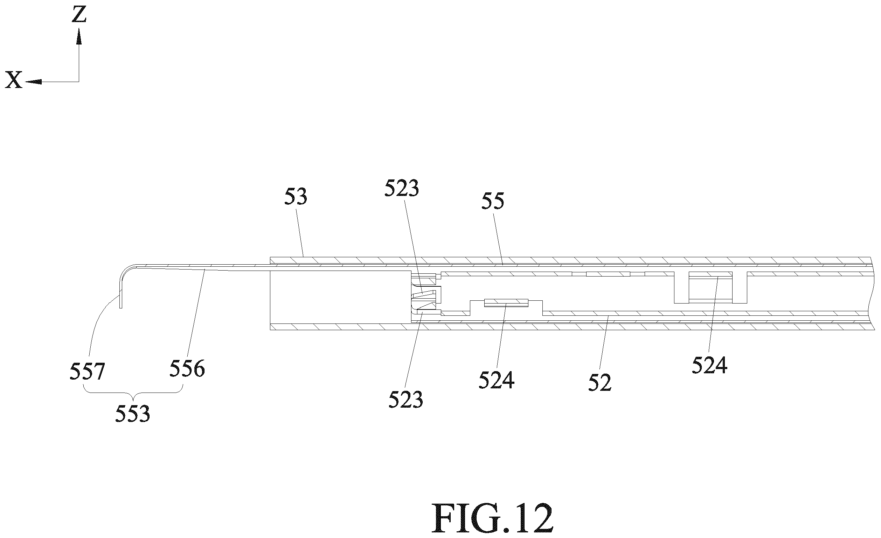

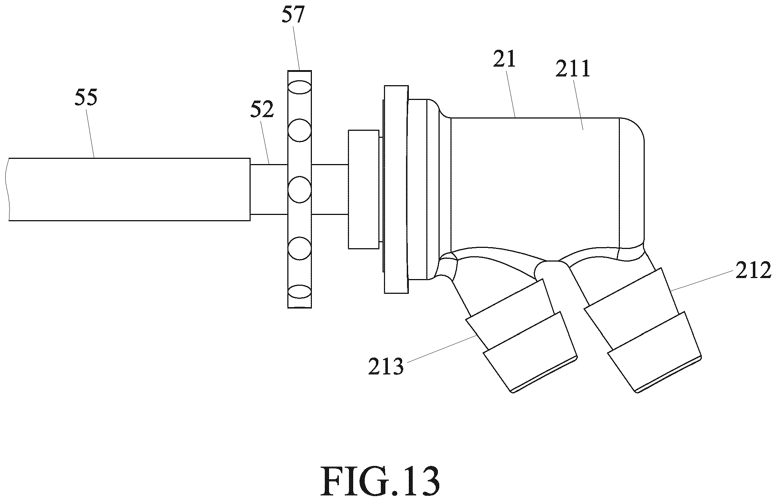

With reference to FIGS. 12 and 13, in a second embodiment, the third cutter portions 555 are dispensed with, and the shape of the second cutter portions 524 is different from that of the first embodiment. The suction irrigation cannula 52 is shown in FIG. 12 not to directly contact with the outer tube 55. It is comprehensive that a portion of the suction irrigation cannula 52 is contact with the outer tube 55 to pass electric current therein through the outer tube 55.

In this embodiment, the outer tube 55 is not rotatable, and a rotatable disc 57 is further disposed securely to the rear end of the suction irrigation cannula 52. Through rotation of the rotatable disc 57, the suction irrigation cannula 52 is rotated relative to the outer tube 55 to perform a breaking up procedure.

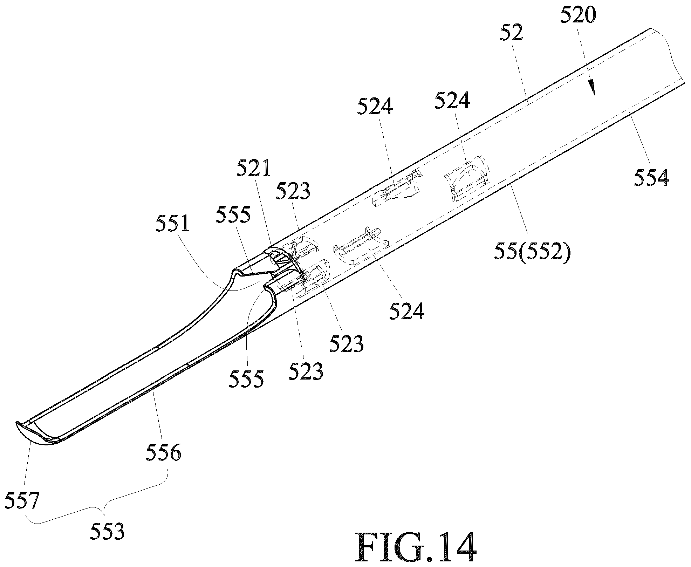

With reference to FIG. 14, in a third embodiment, the hemostatic portion 553 of the outer tube 55 has a curve front tip end 557 with a larger surface area for prompting hemostasis effect.

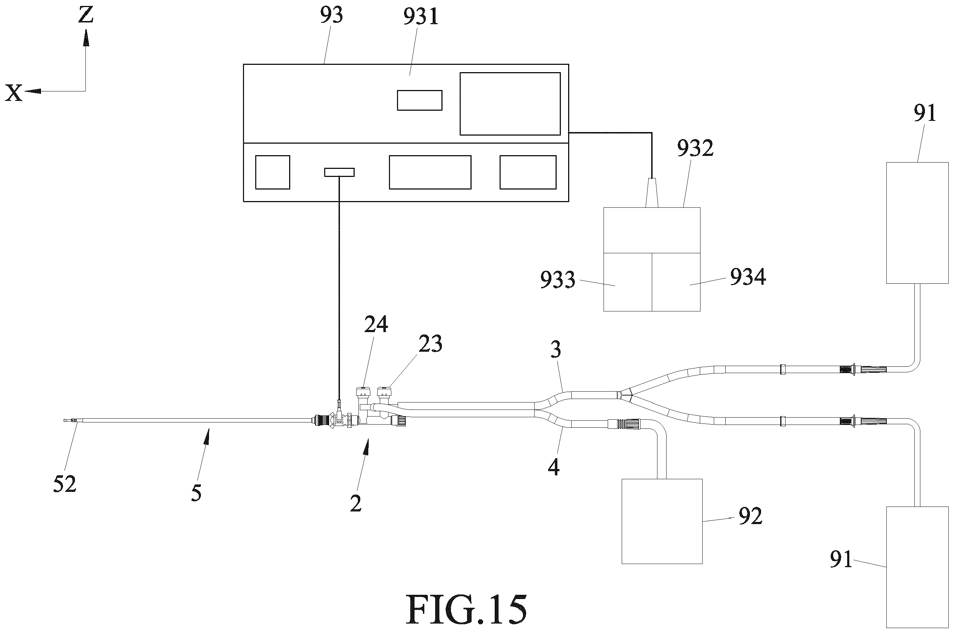

With reference to FIGS. 15 to 17, in a fourth embodiment, the electrocautery suction irrigation tubing device is utilized with two external irrigator devices 91, an external suction device 92 and an external electrocautery machine 93 for surgical processes. The electrocautery machine 93 includes a machine body 931 and a pedal control switch 932 electronically connected with the machine body 931. The pedal control switch 932 has a left pedal 933 and a right pedal 934. The electrocautery suction irrigation tubing device includes a handle module 2, an irrigation hose 3 removably mounted on the handle module 2, a suction hose 4 removably mounted on the handle module 2, and an electrocautery suction irrigation module 5 mounted on the handle module 2 along the front-rear direction (X) and adapted to be electronically connected with the electrocautery machine 93.

The handle module 2 includes a communicating tube 21, and a first actuator button 23 and a second actuator button 24 which are operable and mounted on an upper side of the communicating tube 21.

The communicating tube 21 has a main tube body 211 which extends in the front-rear direction (X) to define a main passage 201, a first tube body 212 which extends upwardly and vertically from the main tube body 211 to define a first passage 202, and a second tube body 213 which is disposed forwardly of the first tube body 212 and which extends upwardly and vertically from the main tube body 211 to define a second passage 203.

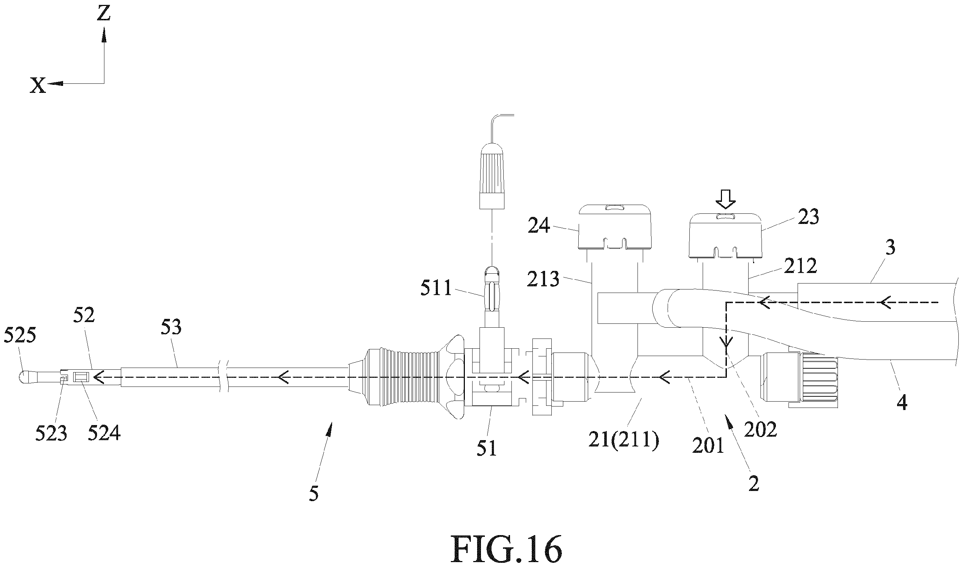

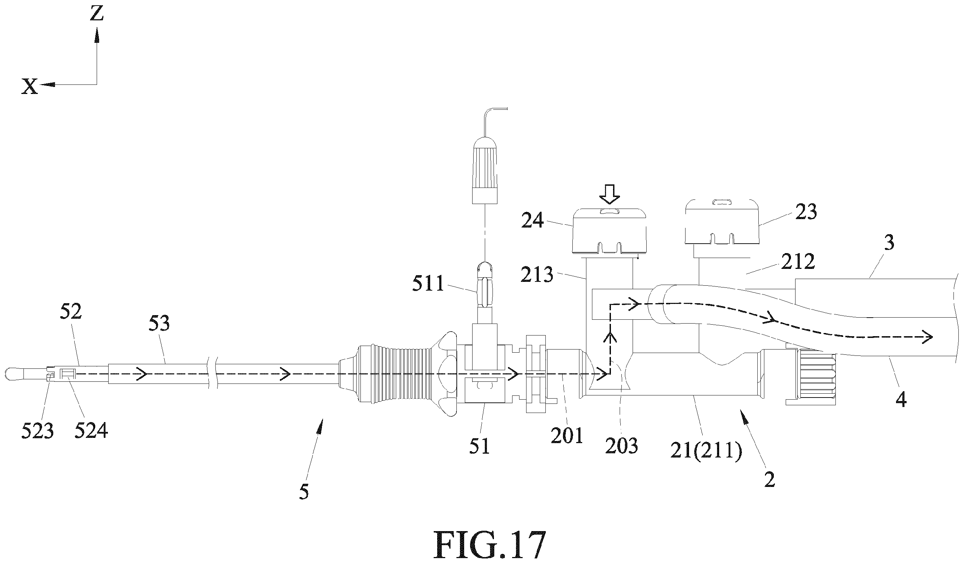

The first actuator button 23 is disposed on an upper end of the first tube body 212 to control fluid communication of the first passage 202 with the main passage 201. The second actuator button 24 is disposed on an upper end of the second tube body 213 to control fluid communication of the second passage 203 with the main passage 201. In this embodiment, each of the first and second actuator buttons 23, 24 is in the form of a press button. FIG. 16 illustrates a state when the first actuator button 23 is pressed, and the first passage 202 is in fluid communication with the main passage 201. FIG. 17 illustrates a state when the second actuator button 24 is pressed, and the second passage 203 is in fluid communication with the main passage 201.

With reference to FIGS. 15 and 16, one end of the irrigation hose 3 is removably mounted on a side of the first tube body 212 and is in fluid communication with the first passage 202. The other end of the irrigation hose 3 is of a Y-shape and is connected with the two irrigator devices 91. When the first actuator button 23 is pressed, saline solution from one irrigator device 91 enters the irrigation hose 3 and flows through the first passage 202 to the main passage 201. Saline solution will be applied from the other irrigator device 91 when no saline solution is left in this irrigator device 91.

With reference to FIGS. 15 and 17, one end of the suction hose 4 is removably mounted on a side of the second tube body 213 and is in fluid communication with the second passage 203. The other end of the suction hose 4 is connected with the suction device 92. When the second actuator button 24 is pressed, blood, body tissues and blood coagulum from the surgical site are removed by the negative pressure generated by the suction device 92 to enter the main passage 201, and flow through the second passage 203, the suction hose 4 to the suction device 92.

The electrocautery suction irrigation module 5 includes a tubular joint 51 which is connected with the communicating tube 21, a suction irrigation cannula 52 which is inserted into the tubular joint 51 and in communication with the main passage 201, and an insulation sleeve 53 which surrounds the suction irrigation cannula 52. The tubular joint 51 has a metal piece 511 which is electronically connected with the machine body 931 of the electrocautery machine 93 and which is in electrical contact with the suction irrigation cannula 52 to permit electric current to pass through the suction irrigation cannula 52 through the metal piece 511 from the electrocautery machine 93. Specifically, when treading the left pedal 933, large electric current is applied to the electrocautery suction irrigation module 5 from the electrocautery machine 93 such that the suction irrigation cannula 52 can cut body tissues or blood coagulum. When treading the right pedal 934, small electric current is applied to the electrocautery suction irrigation module 5 from the electrocautery machine 93 to perform an electrocoagulation hemostasis procedure.

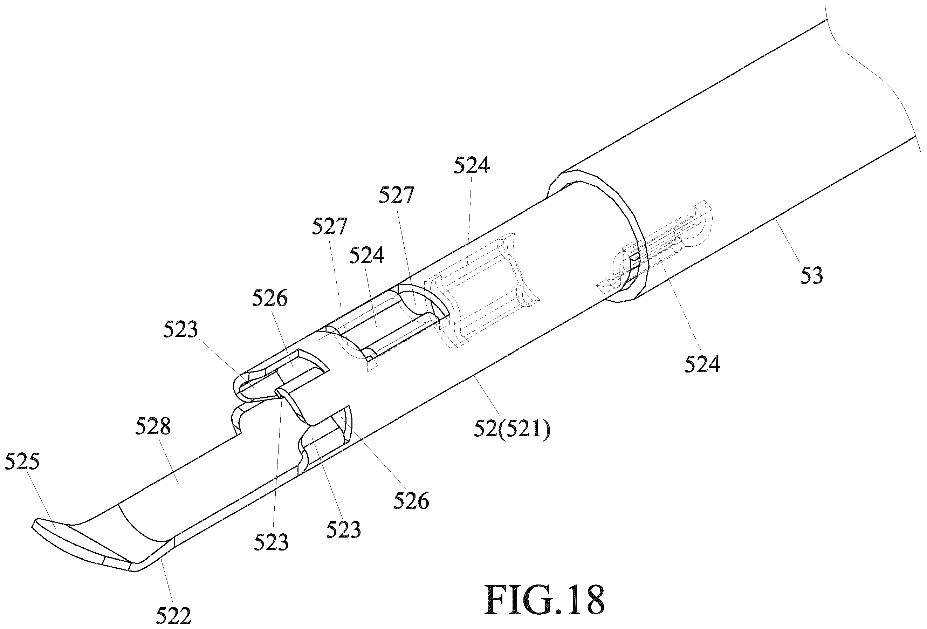

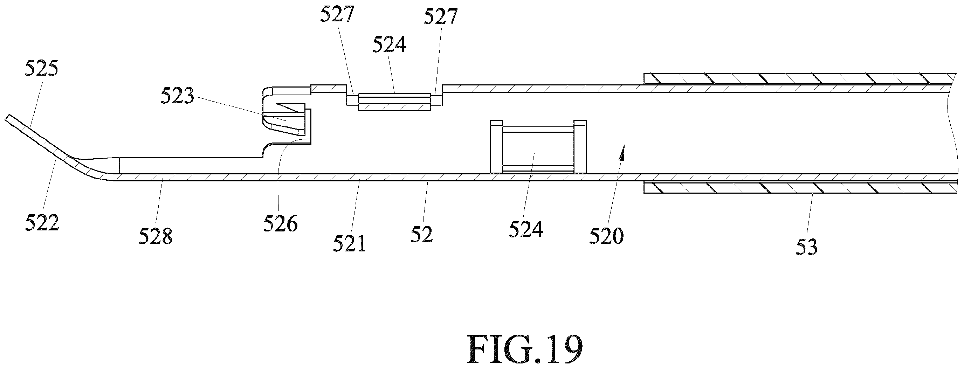

With reference to FIGS. 18 to 20, the suction irrigation cannula 52 has a cannula wall 521 which defines a cannula passage 520, a hemostatic portion 522 which extends forwardly from a front end of the cannula wall 521, a plurality of first cutter portions 523 which are formed on the front end of the cannula wall 521 and which extend in the cannula passage 520, and a plurality of second cutter portions 524 which are formed on the cannula wall 521 and rearwardly of the first cutter portions 523 and which extend in the cannula passage 520. The cannula passage 520 is in fluid communication with the main passage 201.

The hemostatic portion 522 has a front tip end 525 away from the first cutter portions 523, and an elongated section 528 interconnecting the front tip end 525 and the cannula wall 521. The front tip end 525 is in the form of a flat plate, and extends and is inclined forwardly and upwardly from the elongated section 528. The front tip end 525 of the hemostatic portion 522 is attached to the bleeding site. An electrocoagulation hemostasis procedure is performed through the resistance and heat generated as a result of the front tip end 525 contacting the body tissues. The front tip end 525 of this embodiment is adapted to perform an electrocoagulation hemostasis procedure for a large hemostatic area.

The first cutter portions 523 are formed at the same axial position of the suction irrigation cannula 52, and are angularly spaced apart from each other in a circumferential direction of the suction irrigation cannula 52. The first cutter portions 523 are bent from the cannula wall 521 toward the cannula passage 520. In making the suction irrigation cannula 52 in an integrally formed manner, a notch 526 is formed between two adjacent ones of the first cutter portions 523. The second cutter portions 524 are more remote from the hemostatic portion 522 than the first cutter portions 523.

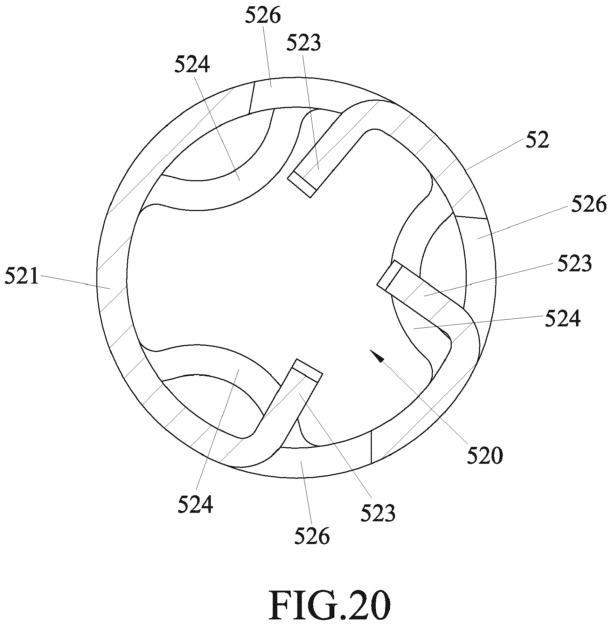

The second cutter portions 524 are spaced apart from each other in an axial direction of the suction irrigation cannula 52, and are angularly spaced apart from each other in a circumferential direction of the suction irrigation cannula 52. As shown in FIG. 20, the second cutter portions 524 are formed on the cannula wall 521 at equal distances, e.g., 120 degrees. Each second cutter portion 524 is curved and concaved from the cannula wall 521 radially and inwardly toward a center of the cannula passage 520. In making the suction irrigation cannula 52 in an integrally formed manner, two notches 527 are respectively formed in front and rear ends of each second cutter portion 524.

During the fluid suction mode when the second actuator button 24 is pressed, the pedal control switch 932 is selectively operated to actuate an electrocauterization procedure or an electrocoagulation hemostasis procedure. Specifically, when treading the left pedal 933, electric current is applied to pass through the suction irrigation cannula 52 such that body tissues or blood coagulum flowing through the first cutter portions 523 and the second cutter portions 524 contact the suction irrigation cannula 52 to generate resistance and heat, which heat and cut the body tissues or blood coagulum into smaller pieces so as to smoothly pass through the passages to reduce blockage in the passages. Once bleeding occurs during the surgery, the front tip end 525 is attached to the bleeding site, and the right pedal 934 is treaded, an electrocoagulation hemostasis procedure is performed.

Therefore, the suction irrigation cannula 52 of this embodiment is operable to perform an electrocautery function. That is, with the first cutter portions 523 and the second cutter portion 524, during the fluid suction mode, electric current is applied to the suction irrigation cannula 52 to heat and cut body tissues or blood coagulum into smaller pieces so as to smoothly pass through the passages to reduce blockage in the passages.

In the description above, for the purposes of explanation, numerous specific details have been set forth in order to provide a thorough understanding of the embodiments. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

## Claims

1. An electrocautery suction irrigation tubing device comprising:
a handle module (2) including a communicating tube (21) and a first actuator button (23) and a second actuator button (24), said communicating tube (21) defining a main passage (201), a first passage (202) which is controlled by said first actuator button (23) to be in fluid communication with said main passage (201), and a second passage (203) which is controlled by said second actuator button (24) to be in fluid communication with said main passage (201); and
an electrocautery suction irrigation module (5) connected with said handle module (2), and including a suction irrigation cannula (52) which is connected and communicated with said communicating tube (21), **characterized in that** said suction irrigation cannula (52) is made of a metal material and integrally formed to be a single piece for passing of electric current, and has a cannula wall (521) which defines a cannula passage (520), and at least one first cutter portion (523) which is formed on said cannula wall (521) and which extends in said cannula passage (520).

2. The electrocautery suction irrigation tubing device of claim 1, **characterized in that** said electrocautery suction irrigation module (5) further includes an outer tube (55), said outer tube (55) being made of a metal material and integrally formed to be a single piece for passing of electric current, and being sleeved around said suction irrigation cannula (52), said outer tube (55) having a hemostatic portion (553) which extends away from said handle module (2) and beyond said suction irrigation cannula (52), said outer tube (55) being rotatable relative to said suction irrigation cannula (52).

3. The electrocautery suction irrigation tubing device of claim 2, **characterized in that** said suction irrigation cannula (52) further has at least one second cutter portion (524) which is formed on said cannula wall (521) and which is bent and extends in said cannula passage (520), said second cutter portion (524) being more remote from said hemostatic portion (553) than said first cutter portion (523).

4. The electrocautery suction irrigation tubing device of one of claim 2 and claim 3, **characterized in that** said outer tube (55) further has a sleeve section (554) which is sleeved around said suction irrigation cannula (52), and at least one third cutter portion (555) which interconnects said sleeve section (554) and said hemostatic portion (553) and which is bent and extends radially and inwardly toward an inner tubular surface (551) of said outer tube (55).

5. The electrocautery suction irrigation tubing device of claim 4, **characterized in that** said electrocautery suction irrigation module (5) further includes a tubular joint (51) which is connected with said communicating tube (21) and in which said outer tube (55) is securely inserted, and a movable member (54) which surrounds said tubular joint (51), said tubular joint (51) having at least one first engaging feature (515), said movable member (54) having at least one second engaging feature (546) which is engageable with said first engaging feature (515) to bring said tubular joint (51) and said outer tube (55) to rotate relative to said suction irrigation cannula (52) when said movable member (54) is rotated relative to said handle module (2).

6. The electrocautery suction irrigation tubing device of one of claims 2, 3 and 4, **characterized in that** said electrocautery suction irrigation module (5) further includes an insulation sleeve (53) which surrounds said outer tube (55) and which is movable relative to said outer tube (55) between an electrocautery state, where at least a portion of said hemostatic portion (553) of said outer tube (55) projects from said insulation sleeve (53), and a suction irrigation state, where said hemostatic portion (553) is covered and concealed by said insulation sleeve (53).

7. The electrocautery suction irrigation tubing device of claim 6, **characterized in that** said electrocautery suction irrigation module (5) further includes a tubular joint (51) which is connected with said communicating tube (21) and said outer tube (55), and a movable member (54) which is securely connected with said insulation sleeve (53) and which is movable relative to said tubular joint (51), said movable member (54) being operated to bring said insulation sleeve (53) to move relative to said tubular joint (51) and said outer tube (55) to move said insulation sleeve (53) between the electrocautery state and the suction irrigation state.

8. The electrocautery suction irrigation tubing device of claim 7, **characterized in that** said movable member (54) has an outer surrounding wall (542) which defines a receiving hole (541) therein, and an inner tubular wall (543) which is radially spaced apart from said outer surrounding wall (542) and which defines an inner hole (544) therein, said tubular joint (51) being disposed in said receiving hole (541), said insulation sleeve (53) being securely connected with said inner hole (544) to be coaxially aligned with said tubular joint (51).

9. The electrocautery suction irrigation tubing device of one of claims 2, 3, 4 and 6, **characterized in that** said handle module (2) includes a handgrip (25) and a switch button (26) operably mounted on said handgrip (25), said switch button (26) being operated to control the passing of electric current into said outer tube (55) and said suction irrigation cannula (52).

10. The electrocautery suction irrigation tubing device of claim 9, **characterized in that** said handgrip (25) has a horizontal upper portion (251) on which said communicating tube (21) is disposed, and a gripping portion (252) which extends downwardly from and is inclined relative to said horizontal upper portion (251), said electrocautery suction irrigation module (5) further including a movable member (54) which is rotatably sleeved around said horizontal upper portion (251) and in which said suction irrigation cannula (52) and said outer tube (55) are engaged such that rotation of said movable member (54) relative to said horizontal upper portion (251) drives one of said suction irrigation cannula (52) and said outer tube (55) to rotate relative to the other one of said suction irrigation cannula (52) and said outer tube (55).

11. The electrocautery suction irrigation tubing device of claim 2, **characterized in that** said hemostatic portion (553) of said outer tube (55) has a curve tip end (557).

12. The electrocautery suction irrigation tubing device of claim 1, **characterized in that** said suction irrigation cannula (52) further has a hemostatic portion (553) which extends forwardly from said cannula wall (521) in a front-rear direction.

13. The electrocautery suction irrigation tubing device of claim 12, **characterized in that** said suction irrigation cannula (52) further has at least one second cutter portion (524) which is formed on said cannula wall (521) and which extends in said cannula passage (520), said second cutter portion (524) being more remote from said hemostatic portion (553) than said first cutter portion (523).

14. The electrocautery suction irrigation tubing device of claim 1, **characterized in that** said suction irrigation cannula (52) has a plurality of said first cutter portions (523), said first cutter portions (523) being formed at the same axial position of said suction irrigation cannula (52), and being angularly spaced apart from each other in a circumferential direction of said suction irrigation cannula (52).

15. The electrocautery suction irrigation tubing device of claim 13, **characterized in that** said suction irrigation cannula (52) has a plurality of said second cutter portions (524), said second cutter portions (524) being spaced apart from each other in an axial direction of said suction irrigation cannula (52), and being angularly spaced apart from each other in a circumferential direction of said suction irrigation cannula (52).
